Europäisches Patentamt

(19) European Patent Office (11) Numéro de publication: **0 038 765**

Office européen des brevets **B1**

(12) **FASCICULE DE BREVET EUROPEEN**

(45) Date de publication du fascicule du brevet: 02.09.87

(21) Numéro de dépôt: 81400634.2

(22) Date de dépôt: 22.04.81

(51) Int. Cl.⁴: **C 12 N 15/00, C 12 P 21/02, A 61 K 39/29**

(54) Vaccin contre l'hépatite virale B, procédé et cellules eucaryotes transformées pour la production de ce vaccin.

(30) Priorité: 22.04.80 FR 8009041
09.12.80 FR 8026132

(43) Date de publication de la demande:
28.10.81 Bulletin 81/43

(45) Mention de la délivrance du brevet:
02.09.87 Bulletin 87/36

(84) Etats contractants désignés:
BE CH DE GB IT LI LU NL SE

(56) Documents cités:
EP-A-0 013 828
EP-A-0 020 251
WO-A-81/00577

NATURE, vol. 280, 30 aou7t 1979, McMillan
Journals Ltd., Basingstoke, P. VALENZUELA et
al.: "Nucleotide sequence of the gene coding
for the major protein of hepatitis B virus
surface antigen", pages 815-819

(73) Titulaire: **INSTITUT PASTEUR**
25-28, rue du Docteur Roux
F-75724 Paris Cédex 15 (FR)
(73) Titulaire: **INSTITUT NATIONAL DE LA SANTE ET DE LA RECHERCHE MEDICALE**
101, rue de Tolbiac
F-75654 Paris Cedex 13 (FR)

(72) Inventeur: **Tiollais, Pierre**
16, rue de la Glacière
F-75013 Paris (FR)
Inventeur: **Chany, Charles**
19, rue Emile Dubois
F-75014 Paris (FR)
Inventeur: **Dubois, Marie Françoise**
93, boulevard de la Reine
F-78000 Versailles (FR)
Inventeur: **Pourcel, Christine**
162, rue de la Convention
F-75015 Paris (FR)
Inventeur: **Louise, Anne**
186, avenue Jean Jaurès
F-75019 Paris (FR)

(74) Mandataire: **Gutmann, Ernest et al**
S.C. Ernest Gutmann - Yves Plasseraud 67,
boulevard Haussmann
F-75008 Paris (FR)

Courier Press, Leamington Spa, England.

(56) References cited:
NATURE, vol. 281, 25 octobre 1979, McMillan
Journals Ltd., F. GALIBERT et al.: "Nucleotide
sequence of the hepatitis B virus genome
(subtype ayw) cloned in E. coli", pages 646-680

NUCLEIC ACIDS RESEARCH, vol. 7, no. 2, 25
septembre 1979, Information Retrieval Ltd.,
London, GB., P. CHARNAY: "Localization on
the viral genome and nucleotide sequence of
the gene coding for the two major
polypeptides of the Hepatitis B surface antigen
(HBs Ag), pages 335-346

Proc. NATL. ACAD. SCI. USA, vol. 77, no. 1,
janvier 1980, E.C. LAI et al.: "Ovalbumin is
synthesized in mouse cells transformed with
the natural chicken ovalbumin gene", pages
244-248

NATURE, vol. 281, 6 septembre 1979, McMillan
Journals Ltd., N. MANTEI et al.: "Rabbit
beta-globin mRNA production in mouse L cells
transformed with cloned rabbit beta-globin
chromosomal DNA", pages 40-46

NATURE, vol. 279, 28 juin 1979, McMillan
Journals Ltd., M. FRIED et al.: "Infectivity in
mouse fibroblasts of polyoma DNA integrated
into plasmid pBR322 or lambdoid phage DNA",
pages 811-816

CHEMICAL ABSTRACTS, vol. 86, no. 25, 1977,
page 413, réf.: 187460r, Columbus, Ohio, US,
D.L. PETERSON et al.: "Partial amino acid
sequence of two major component
polypeptides of hepatitis B surface antigen"
PROC. NATL. ACAD. SCI. USA, vol. 77 (8), aou7t
1980, M.F. DUBOIS et al.: "Excretion of
hepatitis B surface antigen particles from
mouse cells transformed with cloned viral
DNA", pages 4549-4553

PROC. NATL. ACAD. SCI., USA, vol. 76, no. 8,
aou7t 1979, F. COLBERE-GARAPIN et al.:
"Cloning of the active thymidine kinase gene of
herpes simplex virus type 1 in Escherichia coli
K-12", pages 3755-3759

NATURE, vol. 279, 3 Mai 1979, McMillan
Journals Ltd., C.J. BURRELL et al.: "Expression
in Escherichia coli of hepatitis B virus DNA
sequences cloned in plasmid pBR322", pages
43-47

NATURE, vol. 282, 6 décembre 1979, McMillan
Journals LTD., M. PASEK et al.: "Hepatitis B
virus genes and their expression in E.coli",
pages 575-579

**0 038 765**

**Description**

L'invention est relative à une préparation contenant des antigènes possédant des propriétés immunologiques, notamment antigéniques, caractéristiques des virus de diverses formes d'hépatites, telles que l'hépatite virale B ou diverses autres formes d'hépatites telles que celles qui sont connues pour se développer chez certains sujets à la suite de transfusions sanguines, par exemple les hépatites dites "non A" ou "non B". L'invention concerne plus particulièrement des préparations de ce type, qui sont caractérisées par une pureté élevée, l'absence de protéines d'origine humaine et, lorsqu'il s'agit de préparations ayant des propriétés antigéniques analogues à celles des antigènes HBs, exemptes de particules de Dane. Elle est également relative à un procédé de production de ces antigènes.

On sait que certaines des propriétés antigéniques spécifiques du virus de l'hépatite doivent être attribuées à un antigène appelé "Antigène HBs" ou "HBsAg", essentiellement formé par l'enveloppe du virus de l'hépatite virale du type B (HBV) ou particule de Dane. Cet antigène, qui possède des propriétés vaccinantes à l'égard de l'hépatite virale du type B est, à l'heure actuelle, essentiellement obtenu à partir d'échantillons de sérum humain. Cependant on ne dispose pas à ce jour d'autres sources d'approvisionnement en HBsAg, en raison des caractéristiques particulières du virus de l'hépatite B (HBV). Il semble n'être capable d'infecter que l'homme, le chimpanzé et, peut-être, un nombre réduit d'autres primates. Il n'a pas été possible jusqu'à ce jour de la propager *in vitro* dans des cultures de cellules. On connaît certes des lignées d'hépatocarcinomes qui synthétisent de l'HBsAg. L'utilisation chez l'homme de cellules cancéreuses pour la production de particules à caractère vaccinant se heurte à des objections bien compréhensibles. L'utilisation en thérapeutique préventive des antigènes HBs d'origine humaine n'est cependant pas dépourvue de risques sérieux. En effet, ils proviennent en général de personnes qui ont été exposées au virus de l'hépatite virale B, de sorte que la présence de particules de Dane, quelquefois encore hautement infectieuses, dans les préparations d'antigène d'HBsAg d'origine sérique ne peut pas toujours être complètement exclue, même dans le cas de préparations extrêmement purifiées. Les teneurs des préparations même hautement purifiées d'antigène HBsAg de l'état de la technique en protéines sériques ou autres composants éventuellement antigéniques, susceptibles d'induire chez les sujets traités des réactions immunitaires néfastes, ne sont pas négligeables, en raison de leur teneur initiale très faible en HBsAg vis-à-vis des protéines du sérum initial (par exemple de l'ordre de 50 µg d'HBsAg vis-à-vis de 80 mg de protéine dans 1 ml de sérum).

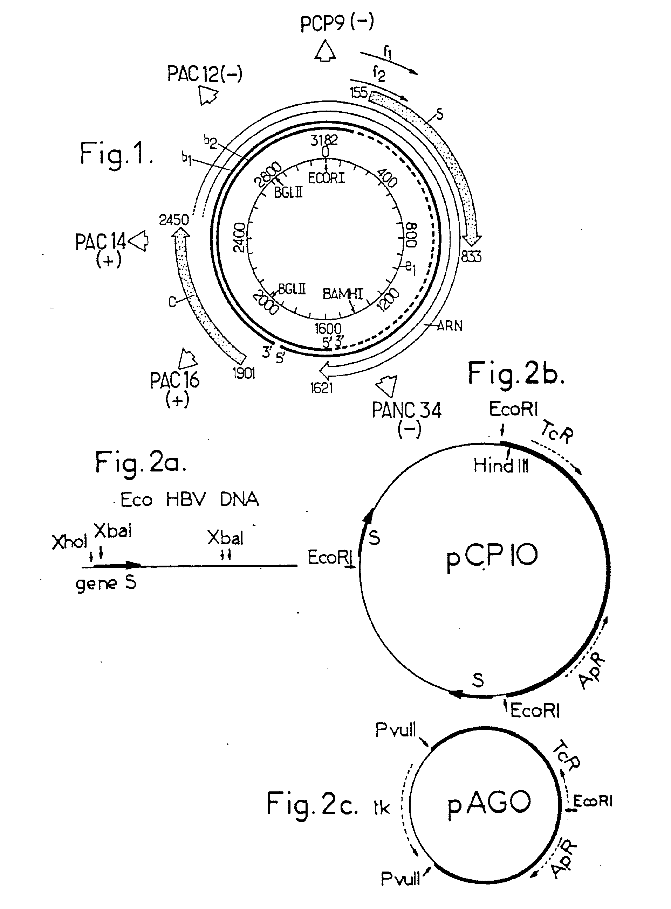

Malgré les difficultés rencontrées pour disposer de quantités suffisantes de virus, il a quand même été établi que le génome du virus de l'hépatite B est formé d'une molécule d'ADN circulaire partiellement à simple brin, dont le brin le plus long comporte de l'ordre de 3200 nucléotides (Summers J. et coll. (1975) Proc. Nat. Sci. U.S.A. *72*, 4597—4601). Au surplus, il a été possible de localiser le gène codant la partie de la protéine de l'antigène HBsAg, qui est responsable des propriétés immunologiques de l'enveloppe du virus de l'hépatite B. La position de ce gène, dénommé "gène S" résulte notamment de la carte schématique du génome de la particule de Dane, qui est donnée dans la figure 1 des dessins.

La figure 1 comprend une carte schématique de l'ADN du génome de la particule de Dane. Cet ADN comporte deux brins $b_1$ et $b_2$, le plus court d'entre eux ($b_2$) étant normalement dépourvu de la partie représentée par une ligne en trait interrompu dans le dessin.

Il est connu que cet ADN ne comporte qu'un seul site EcoRI.

La flèche $f_1$ donne le sens de la numérotation des nucléotides, dont est composé le brin le plus long $b_1$, et la flèche $f_2$ donne le sens de la transcription du gène S par la machinerie cellulaire des cellules envahies par le virus de l'hépatite B.

Le site EcoRI peut donc être numéroté O, ou 3182 (dans le cas des virus de l'hépatite B appartenant au sérotype 3182) (Nature, 1979, vol. 281, p. 646—650).

Le cercle concentrique intérieur $e_1$ donne l'échelle en nombres de nucléotides. Ce cercle permet de préciser les positions de certaines des parties de cet ADN. Les nombres 3', 5' et 5', 3' à la partie inférieure de la carte visent les extrémités terminales porteuses de mêmes nombres dans les représentations conventionnelles des extrémités des chaînes d'acide nucléique.

Au surplus, le gène codant pour la partie de la protéine de l'antigène HBsAg a été localisé. La position de ce gène, dénommé "gène S", est schématisée par la flèche S sur la figure. Le "gène S" est essentiellement porté par le fragment du brin le plus long $b_1$ situé entre les positions nucléotidiques 155 et 833 de la carte schématique de la figure dans le sens de la transcription du gène S.

Le brin le plus court ($b_2$) du génome de la particule de Dane peut être "réparé" *in vitro* en présence de nucléotides précurseurs et d'une polymérase, par exemple selon la technique de T. A. Landers et coll., J. Virol., 23, 1977, p. 368—376. Les génomes ainsi réparés des virus de l'hépatite virale (ou tout ADN susceptible de coder pour les mêmes séquences d'acides aminés) seront, ci-après, désignés par l'abréviation DNA HBV.

L'invention a donc pour but de fournir des procédés tels qu'ils ont été spécifiés plus haut, qui soient applicables d'une façon générale à l'étude et à l'expression dans des cellules eucaryotes de tout ou partie de génomes entiers des virus responsables des différentes hépatites virales, plus particulièrement des DNA HBV. Elle a également pour but la production de vecteurs modifiés permettant la mise en oeuvre de ces procédés.

L'invention a enfin plus particulièrement pour but la production de préparations contenant des

3

**0 038 765**

antigènes présentant la même spécificité immunologique que l'HBsAg ou d'un antigène analogue de haute pureté, comme il a été indiqué plus haut, à partir d'un système autre que le sérum humain, système qui est à la fois reproductible et stable (ou présentant un certain nombre de caractères déterminés qui peuvent faire l'objet d'une surveillance permamente).

Le procédé selon l'invention pour obtenir une culture de cellules eucaryotes compétentes, susceptibles d'exprimer un ADN de virus, choisis parmi ceux qui sont responsables d'une hépatite, la compétence des cellules eucaryotes susceptibles d'être mises en oeuvre pouvant être appréciée par la capacité qui peut leur être conférée, après leur transformation avec un vecteur, contenant une séquence tandem comprenant deux DNA HBV de secréter une protéine immunogène capable d'induire *in vivo* la production d'anticorps actifs contre le virus entier de l'hépatite virale B, est caractérisé par l'opération qui consiste à transformer cette culture de cellules eucaryotes avec un vecteur, plus particulièrement un plasmide, contenant lui-même une séquence d'insertion comportant elle-même au moins la partie de l'ADN viral codant pour une protéine immunogène, capable d'induire *in vivo* la production d'anticorps actifs à l'égard du virus entier, ainsi que le promoteur dudit virus, sous le contrôle duquel s'effectuent la transcription et la traduction chez l'hôte normal infecté par ce virus, de la susdite partie d'ADN viral.

Dans cette description on utilise pour la comodité du langage, l'expression "séquence tandem", pour désigner une séquence d'insertion comprenant deux DNA HBV orientés dans la même direction de transcription et dans laquelle la queue de l'un est reliée à la tête de l'autre.

Dans un premier mode de mise en oeuvre préféré du procédé selon l'invention appliqué à la production d'une culture de cellules eucaryotes exprimant le gène S du génome du virus de l'hépatite B, la susdite séquence d'insertion est formée par ladite séquence tandem.

La séquence d'insertion peut aussi être formée de deux fragments de DNA HBV, normalement contenus dans DNA HBV de part et d'autre de son site EcoRI, et respectivement orientés dans la même direction de transcription, l'un de ces fragments de DNA HBV contenant la séquence de nucléotides du gène S, qui est apte à coder pour le polypeptide responsable des propriétés immunologiques de l'antigène HBs et le second desdits fragments étant suffisamment long pour qu'il soit susceptible d'inclure le promoteur du gène S, la queue de l'un de ces fragments étant reliée à la têtre de l'autre fragment au niveau d'un site EcoRI.

Il va de soi que certaines parties non essentielles de la séquence d'insertion peuvent être délétées. Une séquence d'insertion appropriée dérivée de DNA HBV contient le fragment de nucléotides du gène S codant pour le polypeptide responsable HBs, cette séquence étant précédée vis à vis du sens de la transcription, d'un fragment de DNA HBV suffisamment long pour qu'il soit susceptible d'inclure le promoteur du gène S, en amont du précédent dans le sens de la transcription, ces deux fragments étant normalement situés de part et d'autre du site EcoRI de DNA HBV.

En d'autres termes, cette séquence d'insertion comprend notamment, si l'on se réfère à la figure I, d'une part, le sous-fragment compris entre les positions nucléotiques 833 à 0 (sens inverse de $f_1$), lorsque ce sous-fragment comprend le gène S dans son entier et, d'autre part, un sous...fragment compris entre la position 3182 et une position correspondant à un nombre suffisamment éloigné de cette position 0, dans le sens inverse de la transcription, pour que le promoteur susdit soit susceptible d'y être inclus.

Il s'avère que le dernier sous-fragment devra normalement comprendre une séquence TATATAA, laquelle se trouve normalement en amont du début du gène S, à une distance correspondant à un nombre de nucléotides de l'ordre de 235. Plus particulièrement encore, la séquence d'insertion comprendra une partie supplémentaire du DNA HBV se situant en amont du site BglI, lui-même au niveau du nucléotide 2840.

Conformément à une caractéristique supplémentaire de l'invention, la séquence d'insertion comprend également en outre un sous-fragment en aval du gène S dans le sens de la transcription, ce fragment s'étendant normalement dans le sens de la transcription, au-delà du site BamHI situé entre le gène S et le gène C, de DNA HBV.

Une séquence d'insertion préférée comprend la totalité du gène codant pour l'ARN messager correspondant à l'antigène HBs. La position relative de cet ARN messager est représentée dans la figure 1 par la flèche ARN, dont le début coïncide sensiblement avec la position du nucléotide 2800, si l'on se réfère à la carte de la figure 1, et qui se termine entre les sites BamH 1 et BGIII 1986.

Une séquence d'insertion préférée susceptible d'être mise en oeuvre dans le procédé selon l'invention est donc caractérisée en ce qu'elle comporte à la fois le gène S et le gène codant pour l'ARN messager correspondant à l'antigène HBs. Comme il résultera de la suite de cette description, cette séquence est de préférence dépourvue de toute partie du gène codant pour l'antigène HBc, suffisant à s'exprimer sous forme d'une protéine ayant les propriétés immunogènes de l'antigène HBc.

L'invention concerne également l'application des vecteurs modifiés par les séquences d'insertion susindiquées à la fabrication des protéines susceptibles d'être codées par les fragments d'ADN contenus dans la séquence d'insertion susdite, par transformation d'une culture de cellules eucaryotes, caractérisée en ce qu'elle comprend la récupération des protéines excrétées à partir du milieu de culture desdites cellules.

Dans la suite de cette description, il sera fait référence plus particulièrement à DNA HBV. Il est néanmoins entendu que toutes les techniques qui sont décrites sont également applicables aux ADN ou génomes entiers correspondants des autres virus, responsables des autres types d'hépatites.

- 4

**0 038 765**

De préférence, on se place dans des conditions qui permettent en même temps un marquage des cellules transformées, de façon à rendre leur détection aisée, par exemple en mettant en oeuvre des cellules ou mutants de ces cellules rendues déficientes, à des fins de sélection (déficience naturelle ou induite), en un gène marquer sélectionnable normalement nécessaire à leur croissance, lorsqu'elles sont placées dans certains milieux de culture déterminées, ces cellules étant néanmoins susceptibles de se développer à nouveau dans l'un au moins de ces mêmes milieux de culture, après introduction dans ces cellules d'un gène ou fragment d'ADN homologue, quoique d'origine étrangère (ADN de complémentation), susceptible de compenser ou "complémenter" ladite déficience, le procédé consistant alors, soit à tenter de réaliser la transformation desdites cellules:

— soit avec un seul vecteur contenant, d'une part, la séquence d'insertion étudiée, notamment dérivée de DNA HBV, et, d'autre part, un tel "ADN de complémentation", respectivement inséré dans son propre génome,
— soit avec deux vecteurs (co-transformation ou transformations simultanées),
— l'une de ces vecteurs, de préférence un plasmide, ayant préalablement été modifié par l'insertion dans son génome d'un tel ADN de complémentation;
— l'autre vecteur, de préférence un plasmide aussi, ayant préalablement été modifié par l'insertion dans son génome de la susdite séquence d'insertion,
— et à recueillir, après leur culture dans le milieu susdéfini les colonies qui se sont développées et qui ont été transformées par la séquence d'insertion.

Dans la mesure où le DNA HBV est susceptible d'être exprimé dans la cellule de ladite culture, la séquence tandem (ou contenant plus de deux unités de DNA HBV), ou d'une façon plus générale la séquence d'insertion telle qu'elle a été définie plus haut, doit normalement se comporter comme le ferait l'ADN circulaire correspondant ainsi introduit dans la cellule, en l'absence de tout vecteur.

L'utilisation d'un tel vecteur, notamment plasmide, en lieu et place de l'ADN circulaire lui-même est d'un très grand intérêt en ce que l'on peut disposer de quantités importantes d'un tel vecteur après amplification par clonage dans une bactérie du vecteur préalablement construit *in vitro*.

Le procédé selon l'invention permet donc de tester la capacité d'un ADN circulaire ou d'un génome entier de virus—en particulier des ADN des virus d'hépatites autres que DNA HBV—à s'exprimer dans des cellules eucaryotes, sans qu'il soit nécessaire de se préoccuper de la position exacte du promoteur sous le contrôle duquel est placée la transcription.

Les vecteurs modifiés par les susdites séquences d'insertion, notamment celles dérivées de DNA HBV peuvent être utilisées pour induire l'expression de ces séquences d'insertion dans des cultures de cellules eucaryotes, notamment de souris ou d'origine humaine.

Lorsqu'on utilise, pour le marquage des cellules transformées, un vecteur distinct, notamment un plasmide contenant le marqueur, on a de préférence recours à un plasmide contenant un "ADN de complémentation", tel que le gène de la thymidine-kinase du virus Herpès simplex HSV-1, lequel peut être excisé du génome du virus par des enzymes de restriction spécifiques, tels que BamHI. A ce gène de la thymidine-kinase d'origine virale correspondent, dans de nombreux types de cellules eucaryotes, des gènes homologues propres à diriger la synthèse de la thymidine-kinase (enzyme phosphorylant la thymidine exogène fournie par le milieu de culture).

La possibilité de suppléer à certaines déficiences génétiques de cellules eucaryotes, notamment de souris, par exemple à celles affectant leur gène de la thymidine-kinase endogène, en raison d'une mutation induite ou provoquée, a déjà été décrite par M. Wigler et coll. (Cell. vol. II, 223—232, 1977). Les cellules déficientes, dites TK⁻, sont sélectionnées en raison de leur incapacité de synthétiser la thymidine-kinase dans un milieu tel que celui connu sous le nom de "milieu HAT" (contenant de l'hypoxanthine et de l'aminoptérine en sus de la thymidine). Ce milieu est connu pour n'autoriser l'éventuelle synthèse de thymidine phosphate que par l'intermédiaire d'une voie métabolique dite "voie de sauvetage" (salvation pathway), cette voie impliquant cependant que soit préservée l'intégrité du gène TK des cellules susceptibles de s'y développer. Des cellules TK⁻ deviennent néanmoins capables de se développer dans ce même milieu dès lors qu'elles ont été modifiées par incorporation du gène TK du virus de l'herpès au patrimoine génétique transmissible par ces cellules à leur descendance, comme suite à leurs divisions cellulaires successives. De "TK⁻" qu'elles étaient antérieurement, avant la susdite incorporation, ces cellules sont alors devenues "TK⁺" du fait de la restauration alors constatée de leur capacité de phosphoryler la thymidine dans le susdit milieu et par conséquent de s'y développer.

Bien entendu le fragment d'ADN contenant le gène de la thymidine-kinase peut être remplacé par tout autre ADN de complémentation approprié. A titre d'exemple, d'autres gènes de complémentation qui peuvent être utilisés pour la constitution des vecteurs selon l'invention, on citera celui contenant celui de la dihydrofolate réductase (DHFR), ou d'une façon générale, tout gène de complémentation dont de nombreux exemples existent dans la nature.

L'ADN de complémentation peut naturellement comporter un gène naturel. Il peut aussi être synthétisé, notamment de façon enzymatique, pour copie d'un ARN messager correspondant.

En ce qui concerne la technique de co-transformation elle-même, il est avantageux d'utiliser le vecteur dans lequel est inséré le gène de complémentation et le vecteur dans lequel est inséré le fragment dérivé

5

de DNA HBV dans un rapport élevé supérieur à 100, par exemple de l'ordre de 1000. Plus ce rapport est élevé, plus le nombre de cellules co-transformées par les deux vecteurs à la fois est grand.

Il est avantageux d'avoir recours aux mêmes vecteurs de base pour former les deux types de vecteurs modifiés utilisés pour la co-transformation. Un vecteur de base particulièrement favorable est constitué par le plasmide pBR 322. On pourra en particulier insérer un "DNA HBV tandem" dans son site EcoRI pour former l'un des vecteurs modifiés de co-transformation (pCP10).

Par l'insertion d'un gène tkHSV, soit dans un site Pvull, soit dans un site Bam HI du même plasmide PBR 322, on peut obtenir le second vecteur de co-transformation (pAGO).

En ce qui concerne les conditions de culture, il va naturellement de soi que le milieu préalablement utilisé pour atteindre à un développement suffisant des cultures, doit être remplacé au moment de la transformation on peu après celle-ci par un milieu (tel que le milieu HAT dans le cas où l'ADN de complémentation choisi est un gène de thymidine-kinase) dans lequel les cellules non complémentées sont incapables de se développer.

La réalisation desdites co-transformations, particulièrement lorsque l'on met en jeu des plasmides modifiés préférés susdits dans des fibroblastes de souris, conduit au résultat remarquable que constitue l'excrétion par les cellules co-transformées dans le milieu de culture de particules présentant des propriétés immunologiques caractéristiques des enveloppes du virus de l'hépatite B, notamment en ce qui concerne leur agglutination par des anticorps susceptibles d'agglutiner les antigènes HBsAg naturels, tels qu'isolés des sérums humains. La présence de particules excrétées agglutinables par des anticorps anti-HBsAg peut être détectée par des essais radio-immunologiques classiques, par exemple par immunofluorescence indirecte mettant en jeu des sérums anti-HBsAg et des anti-IgG fluorescents de lapin.

Les quantités formées peuvent être dosées par des tests d'hémagglutination passive directe, de façon en soi connue pour les HBsAg naturels.

Il est également remarquable que les particules excrétées, notamment dans des cultures de cellules de souris, ne contiennent pas de traces détectables d'antigènes HBcAg et HBeAg, détectables par immunofluorescence directe mettant en jeu des anticorps humains fluorescents anti-HBc et anti-HBe/1,2,3. Par mise en contact des cellules co-transformées elles-mêmes avec un sérum anti-HBsAg des granules cytoplasmiques individualisées sont révélées par immunofluorescence indirecte dans la majeure partie des cellules co-transformées. Ces particules peuvent être également repérées après lyse des cellules. Dans la plupart des cas les particules excrétées forment la majeure partie des particules agglutinables par des anticorps anti-HBsAg susceptibles d'être produites.

Les particules formées, qu'elles soient excrétées dans le milieu ou retenues dans le cytoplasme des cellules co-transformées, présentent en core les propriétés qui seront encore mentionnées plus loin dans la description d'exemples.

Il s'est avéré cependant que la transformation de cellules humaines HeLa avec un ADN cloné circularisé d'un virus de l'hépatite B a conduit à l'excrétion par ces cellules non seulement d'antigène HBs, mais également de particules virales complètes contenant de l'antigène HBc dans le milieu de culture (9).

Aussi est-il avantageux, conformément à un perfectionnement supplémentaire de l'invention, d'avoir recours à une séquence d'insertion telle qu'elle a été définie plus haut, dérivée de DNA HBV, dont on a au préalable excisé certaines parties extérieures au gène S du DNA HVB, de façon à n'utiliser que des génomes défectifs qui ne sont plus capables de coder des particules de Dane entières. En particulier, on aura avantageusement recours à une séquence d'insertion dont a été délétée une partie suffisante du gène codant pour l'antigène HBc, pour que la production de ces derniers antigènes par les cellules infectées soit rendue impossible.

Ces séquences d'insertion peuvent par conséquent être utilisées pour modifier des vecteurs qui seront alors aptes à transformer des cellules eucaryotes, qu'il s'agisse de fibroblastes de souris ou de cellules humaines, mais en l'absence de toute possibilité de production d'antigène HBc.

En particulier, l'ADN d'insertion est avantageusement constitué par l'ADN du virus de l'hépatite virale B, dont a été délété le fragment délimité par les extrémités BglII au niveau des nucléotides 1986 et 2425, si l'on se réfère à la carte schématique de la figure 1 à l'intérieur du gène C, dont la position relative vis-à-vis de l'ADN entier a été symbolisée par la flèche C.

On peut également avoir recours à des séquences d'insertion originaires du DNA HBV, dont auront été délétés des fragments plus importants, notamment telles que déjà définies ci-dessus.

Bien entendue, cet ADN d'insertion peut comprendre des délétions supplémentaires, pour autant que celles-ci n'affectent ni la capacité d'expression de la séquence d'insertion, ni les phases de sa traduction. De même, l'invention s'étend à l'usage de toute séquence d'insertion équivalente, qu'il s'agisse de la séquence correspondante d'un ADN viral appartenant à un autre sous-type que celui qui a été envisagé dans le présent texte et repris dans les exemples, ou d'une séquence dont certaines parties auraient été modifiées sans que soit pour autant altérée la capacité des produits d'expression de réagir immunologiquement avec des anticorps contre des antigènes HBs.

Il a ainsi été possible d'obtenir, à partir de cultures de cellules de fibroblastes de souris transformées avec des vecteurs modifiés par des séquences d'insertion, telles que définies ci-dessus, des préparations d'antigènes présentant les caractéristiques immunologiques de l'antigène HBsAg, dépourvues (dans les limites des méthodes de détection disponibles) de toute particule de Dane et des autres antigènes

6

0 038 765

caractéristiques contenus dans ces dernières, en l'absence de composants d'origine humaine, notamment de toute protéine sérique.

En outre, il a été vérifié que les particules excrétées dans les milieux de culture possèdent effectivement des propriétés immunogènes vaccinantes, comme m'atteste leur capacité d'induire la production *in vivo* d'anticorps, analogues aux anticorps anti-HBsAg humains et actifs contre le virus de l'hépatite, plus particulièrement de l'hépatite B, quand elles sont administrées in vivo, notamment chez la souris ou le lapin, selon les protocoles expérimentaux couramment utilisés pour vérifier l'immunogénicité des antigènes HBs naturels, extraits de sérums humains.

L'invention concerne donc également la composition nouvelle utilisable pour la constitution de vaccins, caractérisées en ce qu'elle consiste en des particules contenant des protéines, ayant les propriétés immunogéniques et immunologiques caractéristiques de l'antigène HBsAg, ayant un niveau de pureté total pour ce qui est de l'absence de toute particule de Dane, d'antigène HBc, de tout composant sérique d'origine humaine et d'ADN-polymérase, ces particules ayant été synthétisées par une culture de cellules eucaryotes transformées au préalable par un vecteur contenant le gène S du génome du virus de l'hépatite B.

L'invention concerne encore un produit ayant des propriétés vaccinantes contre le virus de l'hépatite B, caractérisé

— en ce qu'il consiste en des particules ayant des tailles de 18 à 25 mm et une densité permettant leur isolement dans une zone de densité de 1,20 g/ml dans un gradient de densité à base de CsCl.
— en ce que ces particules sont agglutinées par des antigènes qui agglutinent l'anticorps HBsAg de sérum humain;
— en ce que ces particules sont capables d'induire *in vivo* la production d'anticorps actifs contre le virus de l'hépatite B;
— en ce qu'elles ont un degré de pureté totale pour ce qui est des particules de Dane, des antigènes HBc et HBe qui sont totalement absents et
— en ce qu'elles sont exemptes de tout composant humain, notamment de protéines humaines.

En particulier, l'invention concerne les produits dont les particules sont sphériques et qui sont dépourvus d'ADN-polymérase.

L'invention concerne encore plus particulièrement des compositions de vaccins contenant le susdit produit (tel qu'il peut être récupéré à partir des milieux de culture desdites cellules co-transformées ou de lysats de ces dernières), associé à tout véhicule pharmaceutique approprié à la constitution de vaccin actif contre l'hépatite virale, susceptible d'être administré par voie orale ou parentérale.

Elle concerne également les réactifs de laboratoire contenant des doses déterminées de ces antigènes, susceptibles notamment d'être utilisés à titre d'étalons ou de références vis-à-vis desquels peut être apprécié le degré de pureté de préparations contenant des antigènes HBsAg, d'origine naturelle ou non, en ce qui concerne en particulier leurs teneurs relatives en protéines sériques, ou autres antigènes, tels que HBcAg ou HBeAg, etc.

L'invention sera encore davantage illustrée par la description d'exemples de mise en oeuvre des plasmides modifiés (qui font eux-mêmes partie de l'invention) en vue de la fabrication des antigènes ayant des propriétés immunologiques de l'HBsAg. Il sera à cette occasion encore fait référence à la figure 1 déjà mentionnée et aux figures 2a, 2b et 2c, lesquelles représentent des cartes schématiques des plasmides préférés qui ont été mis en oeuvre.

Les nombres entre parenthèses renvoient à la bibliographie ajoutée à la fin de la présente description.

Exemple I
Construction d'un recombinant contenant l'ADN du virus de l'hépatite virale B

200 ng de pBR 322 sont soumis à digestion par l'endonucléase EcoRI et traités avec 2,6 unités de phosphatase alcaline dans 100 nM de Tris-HCl, pH 8, à 60°C pendant 60 minutes. Après deux extractions au phénol, puis trois extractions à l'éther, l'ADN est précipité par l'éthanol. Le résidu solide est dissous dans de l'eau et l'on ajoute à la solution 100 ng de Eco HBV DNA. La ligature est effectuée selon la méthode décrite dans (1).

On procède à une culture de *E. coli* DP 50 dans un milieu de culture constitué par un bouillon L contenant 100 µg/ml d'acide diaminopimélique et 20 µg/ml de thymidine.

Les bactéries sont alors transformées selon la méthode sus-visée par le mélange des recombinants sélectionnés selon leur capacité de résistance à des doses de 100 µg/ml d'ampicilline et 15 µg/ml de tétracycline. 900 colonies sont obtenues. Elles sont toutes testées en vue de la détermination de la présence ou non de HBV DNA par hybridation *in situ*. Le test est positif pour 800 colonies. On recueille les 16 colonies qui induisent un signal d'hybridation d'intensité plus élevée. Les plasmides sont extraits et leur structure est analysée par digestion en présence de EcoRI, Xho I, Hind III et Xba I.

La structure du recombinant obtenu résulte des figures 2a et 2b dans lesquelles ont été schématiquement représentés, d'une part, la structure du gène Eco HBV DNA et, d'autre part, la structure du plasmide modifié pCP10 et telle qu'elle résulte de l'insertion dans le plasmide pBR 322 d'un fragment

7

d'ADN constitué par deux fragments Eco HBV DNA successifs, la tête de l'un étant reliée à la queue de l'autre, au niveau d'un site EcoRI (insertion en tandem tête à queue).

Il s'est avéré que parmi les 16 colonies ci-dessus qui ont été retenues, 14 d'entre elles hébergeaient des plasmides du type pCP 10, dans lesquels les deux Eco HBV DNA se trouvaient insérés selon l'arrangement tandem tête à queue dans les deux directions d'orientation possibles.

C'est en particulier grâce aux digestions avec les endonucléases Hind III et Xba I qu'il a été possible de déterminer les directions d'insertion des fragments d'ADN Eco HBV DNA.

Les intégrations dans le plasmide pCP 10 de deux fragments Eco HBV DNA sont démontrées par la digestion en présence de l'endonucléase Xho I, lesquelles produisent l'excision d'un fragment d'ADN ayant une taille similaire à celle de Eco HBV DNA à partir du plasmide hybride pCP 10. Ce dernier comporte 10.626 paires de bases.

Les positions respectives du gène S dans Eco HBV DNA (figures 2a et 2b) et dans le plasmide hybride pCP 10 sont respectivement représentées par les flèches S dessinées en traits renforcés. Les petites flèches latérales situent les positions relatives de certains des sites de restriction dans les chaînes d'ADN correspondantes. Les positions des facteurs de résistance à l'ampicilline ($Ap^R$) et à la tétracycline ($Tc^R$) sont également représentées de façon schématique.

Les mêmes conventions s'appliquent au plasmide pAGO (figure 2c) qui a été obtenu par insertion dans le site PVU II du plasmide pBR 322 du gène de la thymidine-kinase du virus Herpès simplex HSV-1 (HSV-tk) (2).

Culture et transformation par les susdits plasmides de cellules de souris déficientes en thymidine-kinase ($Ltk^-$)

Des mutantes de cellules de souris LM déficientes en thymidine-kinase ($Ltk^-$) sont cultivées dans le milieu minimum essentiel MEM 0 111 Gibco, le cas échéant en présence de sérum de veau à 10%. Des monocouches confluentes de ces cellules ($2 \times 10^6$ cellules par 25 cm²) dans des flacons de Falcon ont été inoculées avec les ADN correspondant auxdits plasmides, en vue de leur transformation, selon la méthode de Graham et Van Der EB (3) modifiée par Stow et Wilkie (4).

On utilise pour effectuer cette transformation à la fois le plasmide pAGO (linéarisé par l'endonucléase Hind III) et le plasmide pCP10 linéarisé ou non par la même enzyme. Dans tous les essais le rapport moléculaire pAGP/pCP 10 a été de l'ordre de 1/1.000. On a utilisé de l'ADN se sperme de saumon à titre de véhicule pour ajuster la concentration en ADN à au moins 10 µg/ml.

Après la transformation, les cultures cellulaires sont maintenues dans ce milieu contenant en outre 15 µg/ml d'hypoxanthine, 0,1 µg/ml d'aminoptérine et 5 µg/ml de thymidine (milieu sélectif de HAT).

24 heures après la transformation, on ajoute au milieu une solution de HAT concentrée 100 fois, solution concentrée qui est changée une semaine plus trad, puis tous les trois jours.

Après 15 jours de culture en présence des deux types de plasmides (co-transformation) dans le milieu sélectif de HAT, on observe la formation de colonies. 20 jours après la co-transformation, on détecte la production d'HBsAg dans le milieu de culture par des tests radio-immunologiques. Des cultures formées dans les mêmes conditions et transformées à des fins de comparaison avec le seul plasmide pAGO n'induisent aucune production d'HBsAg.

Les colonies $tk^+$ résistantes au milieu HAT sont recueillies avec une pipette Pasteur (20 jours après la co-transformation) et transférées dans des cultures de tissus en microplaques. On effectue des passages des colonies tous les 5 hours et on les maintient sous pression sélective continue dans le milieu de culture HAT.

Cinq des colonies obtenues à partir de la co-transformation en présence de pCP 10 linéarisé et 10 colonies obtenues à partir de la co-transformation des cellules de souris avec le pCP 10 circulaire ont été prélevées et cultivées dans le milieu HAT. Toutes les cultures ont produit de l'HBsAg qui a été libéré dans le milieu de culture externe.

Les quantités d'HBsAg synthétisé sont variables d'une culture à l'autre (dans des limites se trouvant dans un rapport de 1 à 30). Les quantités produites sont stables, même après plusieurs passages desdites cultures.

L'HBsAg peut être récupéré par centrifugation du surnageant des cultures et purifié par centrifugation dans un gradient de densité à base de CsCl. On recueille les HBsAg dans la zone de densité 1,20 g/ml. Ces HBsAg sont complètement neutralisés par une solution de sérum anti-HBsAg (dans un rapport 10/1), après incubation à 37°C pendant une heure, comme détectable par les essais de radio-immunologie.

A l'examen sous le microscope électronique, on observe des particules sphériques ayant des tailles s'échelonnant de 18 à 25 nm (en moyenne 22 nm). Leur morphologie rappelle celle des particules sphériques d'antigènes de 22 nm qui peuvent être isolées à partir de sérum humain. Des structures filamenteuses telles que celles visibles parmi les antigènes extractibles de sérum humain n'ont pas été observées, du moins dans les conditions de l'expérience. Aucune particule de Dane n'a pu être détectée.

Le tableau suivant illustre la capacité de production des cellules de souris $tk^-$ qui ont été co-transformées par les plasmides en question.

Le rapport P/N correspond au rapport du nombre de désintégrations par minute (dpm) mesuré sur le surnageant au nombre de dpm mesuré sur une culture témoin lorsque les dosages de HBsAg sont faits par

8

tests immunologiques 20 jours après la co-transformation des cellules. Un rapport P/N supérieur à 2,1 est considéré comme significatif.

TABLEAU

Co-transformation de cellules Ltk⁻ de souris avec des plasmides pCP10 et pAGO

| Flacon | pAGO linéarisé | pCP10 linéarisé | pCP10 circulaire | ADN de sperme de saumon | Nombre de colonies tk⁺ | P/N |
|--------|----------------|-----------------|------------------|-------------------------|------------------------|-----|
| b | 0,010 | 10 | — | — | 140 | 24 |
| c | 0,005 | — | 5 | 15 | 200 | 16 |
| f | 0,010 | — | — | 15 | 26 | 1 |

Après lyse des cellules préalablement lavées, digestion des protéines en présence de protéinase, extraction et purification de l'ADN cellulaire, dosage de l'ADN cellulaire par la technique de Southern modifiée par Wahl et Coll. (5, 6) en ayant recours à une sonde Eco HBV DNA préparée selon la technique de Weinstok et Coll. (7), il a été constaté que la quantité de HBsAg intracellulaire qui avait été synthétisée correspond approximativement au tiers de l'HBsAg qui avait été excrété dans le milieu de culture.

Dans les conditions de l'expérience, les clones les plus actifs ont produit jusqu'à 150 ng/ml d'HBsAg.

En sus de l'absence déjà mentionnée de particules de Dane, on relève l'absence des antigènes HBcAg et HBeAg au moins à un niveau détectable par immunofluorescence directe mettant en jeu des anticorps fluorescents d'origine humaine anti-HBc et anti-HBe/1,2,3 (8). On n'a pas davantage noté la production d'ADN-polymérase, par mesure de l'activité ADN-polymérase par la méthode de Kaplan et Coll. (tant dans le résidu de centrifugation du surnageant que dans celui du lysat des cellules préalablement transformées).

Par extraction des ADN de poids moléculaire élevé de certains des clones produits, digestion de ces ADN en présence de EcoRI, Hind III et Xho I, fractionnement des fragments obtenus par électrophorèse sur gel d'agarose, transfert sur des filtres de nitrocellulose, hybridation de ces fragments avec des sondes Eco HBV DNA marquées au phosphore 32, il a été établi que plusieurs copies du plasmide pCP 10 pouvaient être intégrées à l'ADN cellulaire. Il a été noté également la présence de dimères d'HBV DNA tant dans les copies de plasmide contenues dans les cellules transformées que parmi les fragments éventuellement intégrés dans les ADN cellulaires.

Il a été également déterminé au moyen d'études cinétiques que dans les conditions expérimentales qui ont été décrites, des particules d'antigène HBsAg peuvent être excrétées dans le milieu par les cellules, à raison de $2 \times 10^4$ à $4 \times 10^4$ particules/cellules/24 heures, soit de 2.000.000 à 4.000.000 de molécules de polypeptides/cellules/24 heures, si l'on suppose que chaque particule contient environ 100 molécules de polypeptides.

Exemple II

Expression dans des cellules eucaryotes de vecteurs modifiés par une séquence d'insertion dérivée de DNA HBV, dont au préalable a été excisée la majeure partie du gène C

1°) Clonage de PCP9 (résultant de l'insertion du plasmide pBR322 linéarisé dans le site EcoRI de DNA HBV)

Le plasmide pBR322 hydrolysé par EcoRI a été traité par la phosphatase alcaline, puis ligaturé en présence d'une quantité équimoléculaire de DNA HBV coupé par EcoRI. La flèche PCP9 symbolise, dans la figure 1, le site d'insertion de pBR322 dans le DNA HBV.

2°) Construction des clones de la série PAC (contenant des fragments DNA HBV coupés par BglII) et PANC (contenant des fragments de DNA HBV coupés par BamHI)

Le plasmide pAGO a été hydrolysé par BamHI et traité par la phosphatase alcaline.

Les fragments HBV ont été obtenus à partir du plasmide PCP10 décrit ci-dessus, après coupure par HindIII et PstI (qui n'ont pas de site dans le DNA HBV), et plus particulièrement, à partir des fragments obtenus, purifiés par électroélution sur un gel d'agarose, et contenant un dimère—ou une séquence "tandem"—de DNA HBV. Ces fragments ont été hydrolysés partiellement, soit par l'enzyme de restriction BglII, soit par l'enzyme BamHI, dans des conditions où seulement deux coupures sont effectuées. Les hydrolysats ont été alors ligaturés en quantités équimoléculaires au plasmide pAGO préalablement coupé par BamHI (cette ligature mettant à profit le fait que les sites BamHI et BglII sont caractérisés par des parties d'extrémités cohésives communes).

Une collection de clones sensibles à la tétracycline a été obtenue et analysée afin d'isoler chaque possibilité d'insertion.

Des clones PAC12, PAC14, PAC16 et PANC34 ont été obtenus. Les flèches accompagnant dans la figure 1 les symboles correspondants, situent les insertions du pBR322 linéarisé dans les sites BamHI ou BglII

9

**0 038 765**

correspondants des séquences de DNA HBV contenues dans les plasmides recombinants obtenus. Ces clones présentaient en outre les caractéristiques suivantes:

PAC12: délétion du fragment BglII 1985—2840;
PAC14: délétion du fragment BglII 1986—2425;
PAC16: insertion du génome de pBR322 dans le site 1986 du DNA HBV;
PANC34: il contient le génome pBR322 dans le site BamHI 1400 du DNA HBV.

Ces clones ont été utilisés pour transformer des cellules LTK⁻ dans les proportions de 2 µg de plasmide pour 2.10⁶ cellules. 4 semaines plus tard, les colonies TK⁺ étaient arrivées à confluence. Le milieu de culture a alors été analysé pour la présence d'HBs par la mise en oeuvre des susdits tests radio-immunologiques.

Les capacités ou non-capacités des divers plasmides recombinants d'exprimer le gène S, sous forme d'antigène HBs excrétés dans le milieu de culture, ont été respectivement exprimés dans la figure 1 par des signes "+" et "−".

L'expression du gène S dans les clones PAC14 et PAC16 et le défaut d'expression dans le clone PAC12 montrent que la transcription du gène S est initiée dans l'ADN viral et plus précisément dans le fragment de restriction BglII 2425—2840. Il existe une séquence TATATAA appelée également "TATATAA Box" située à 72 nucléotides en amont du début de la région "pré-S" qui paraît contrôler la transcription du gène S.

Le fait qu'aucune expression ne soit obtenue avec le clone PANC34, dans les conditions dans lesquelles l'expérience a été réalisée, est peut-être lié au fait que l'ADN codant pour le mRNA s'arrête après le site BamHI.

On retient en particulier le fait que le clone PAC14, qui produit l'HBs, possède une délétion dans le gène C du virus, ce qui élimine le risque de production de particules virales par la cellule transformée, quelle qu'en soit la nature, et permet son utilisation pour la production d'un vaccin.

L'invention fournit donc un produit susceptible d'une pureté inaccessible jusqu'à ce jour. Elle concerne plus particulièrement les préparations formées de particules de protéines ayant les propriétés immunologiques des antigènes HBsAg, essentiellement à l'exclusion de protéines sériques. Elles sont totalement exemptes de particules de Dane décelables par les méthodes usuelles de dosage radio-immunologique. Elles sont au surplus totalement exemptes de protéines, notamment protéines sériques d'origine humaine. Elles sont exemptes d'ADN polyérase.

L'invention conerne également encore les séquences d'ADN d'insertion nouvelles elles-mêmes, telles qu'elles ont été définies plus haut, et les vecteurs, notamment plasmides, contenant lesdites séquences qui sont plus particulièrement caractérisées par le fait qu'elles contiennent le promoteur de la transcription du gène S. Ces vecteurs sont d'un intérét tout particulier en ce qu'ils peuvent éventuellement être modifiés par insertion d'une séquence d'ADN déterminée correspondant à une protéine dont l'expression dans des cellules eucaryotes serait recherchée. En effet, ce vecteur a l'intérêt particulier de n'être pas toxique à l'égard des cellules en cause. Le promoteur n'est pas réprimé, puisque ces cellules permettent l'expression du gène S. En outre, ce vecteur peut permettre l'excrétion des protéines synthétisées directement dans le milieu de culture.

L'une des susdites séquences d'insertion nouvelle peut encore être définie comme contenant, outre le gène S, l'ADN de la région "pré-S" (lequel dans l'ADN du virus de l'hépatite virale est situé immédiatement à l'amont du gène S dans le sens inverse de la transcription, ce gène comportant de l'ordre de 163 nucléotides), le gène de l'ARN messager du gène S du virus de l'hépatite B, le promoteur de l'ADN du virus de l'hépatite B, notamment une séquence TATATAA (T abbréviation de thymine et A abréviation de l'adénine). Elle comprend notamment encore une séquence d'ADN correspondant à une séquence localisée entre les positions nucléotidiques 2425 et 2840 de l'ADN du virus de l'hépatite B.

Sont évidemment incluses dans le cadre de l'invention des séquences comportant plusieurs unités du type sus-décrit, notamment des séquences "tandem".

La capacité des vecteurs modifiés selon l'invention d'être exprimée dans des cellules eucaryotes semble témoigner du fait que la transcription du gène de l'HBsAg est sous le contrôle d'un promoteur virale contenu dans le fragment dérivé d'HBV DNA. Ces vecteurs, tels que le plasmide pCP10, PAC14 ou PAC16, peuvent être eux-mêmes utilisés à titre de vecteurs pour réaliser l'expression dans des cellules eucaryotes d'un ADN étranger préalablement inséré dans lesdits vecteurs. L'avantage des vecteurs modifiés selon l'invention paraît devoir résider dans le fait qu'ils n'induisent pas une lyse des cellules-hôtes. De plus le gène S peut être considéré comme disposant d'une séquence-signal sous le contrôle de laquelle la protéine hybride formée, résultant de l'expression dans les cellules-hôtes de l'ADN étranger préalablement inséré dans le vecteur susdit, serait excrétée dans le milieu de culture. La récupération de cette protéine hybride s'en trouverait évidemment considérablement facilitée.

Bien que le procédé de l'invention ait été décrit dans ce qui précède, principalement dans son application à DNA HBV, il peut s'étendre à tous autres ADN circulaires ou génomes entiers. En particulier il peut être appliqué à l'étude des ADN circulaires des virus "non A", "non B" des hépatites virales correspondantes.

Ci-après la bibliographie relative à l'état de la technique et à laquelle référence est faite dans la description des exemples:

(1) Tiollais, P., Perricaudet, M., Petterson, U. & Philipson, L. (1976), Gene *1*, 49—63.
(2) Colbere-Garapin, F., Chousterman, S., Horodniceanu, F., Kourilsky, P. & Garapin, A. C. (1979), Proc. Natl. Acad. Sci. USA, *76*, 3755—3759.

10

## 0 038 765

(3) Graham, F. L. & Van Der Eb, A. J. (1973), Virology *52*, 456—467.

(4) Stow, N. D. & Wilkie, N. M. (1976), J. Gen. Virol. *33*, 447—458.

(5) Southern, E. M. (1975), J. Mol. Biol. 98, 503—517.

(6) Wahl, G. M., Stern, M. & Stark, G. R. (1979), Proc. Natl. Acad. Sci. USA *76*, 3683—3687.

(7) Weinstock, R., Sweet, R., Weiss, M., Cedar, H. & Axel, R. (1978), Proc. Natl. Acad. Sci. USA *75*, 1299—1303.

(8) Trepo, C., Hantz, L., Vitvitski, L., Chevallier, P., Williams, A., Lemaire, J. M. & Septjian, M. (1978) in Viral Hepatitis, eds. Vyas, G. N., Cohen, S. N. & Schmid, R. (The Franklin Institute Press) pp. 203—209.

(9) Shalomz. Hirschman et Coll., Proc. Natl. Acad. Sci. USA, vol. 77, n° 9, pp. 5507—5511, septembre 1980, "Expression of clone hepatitis B virus DNA in human cell cultures".

**Revendications**

1. Procédé pour obtenir une culture de cellules eucaryotes compétentes, susceptibles d'exprimer un ADN de virus, choisis parmi ceux qui sont responsables d'une hépatite, la compétence des cellules eucaryotes susceptibles d'être mises en oeuvre pouvant être appréciée par la capacité qui peut leur être conférée, après leur transformation avec un vecteur, contenant une séquence tandem comprenant deux DNA HBV, de secréter une protéine immunogène capable d'induire *in vivo* la production d'anticorps actifs contre le virus entier de l'hépatite virale B, est caractérisé par l'opération qui consiste à transformer cette culture de cellules eucaryotes avec un vecteur, plus particulièrement un plasmide, contenant lui-même une séquence d'insertion comportant elle-même au moins la partie de l'ADN viral codant pour une protéine immunogène, capable d'induire *in vivo* la production d'anticorps actifs à l'égard du virus entier, ainsi que le promoteur dudit virus, sous le contrôle duquel s'effectuent la transcription et la traduction chez l'hôte normal infecté par ce virus, de la susdite partie d'ADN viral.

2. Procédé selon la revendication 1, caractérisé en ce que les cellules transformées sont des cellules de souris.

3. Procédé selon la revendication 1, caractérisé en ce que les cellules transformées sont des cellules d'origine humaine.

4. Procédé selon l'une quelconque des revendications 1 à 3, appliqué à la production d'une culture de cellules eucaryotes exprimant le gène S du génome du virus de l'hépatite B, caractérisé en ce que la susdite séquence d'insertion est dérivée de DNA HBV, en ce que la partie de l'ADN viral codant pour la protéine immunogène contient la séquence de nucléotides du gène S codant pour le polypeptide ayant les propriétés immunologiques de l'antigène HBS, cette partie ou fragment de l'ADN viral étant précédé, vis-à-vis du sens de la transcription, d'un fragment de DNA HBV suffisamment long pour qu'il soit susceptible d'inclure le promoteur du gène S.

5. Procédé selon l'une quelconque des revendications 1 à 3, caractérisé en ce que la susdite séquence d'insertion est formée de deux unités de DNA HBV formant une séquence "tandem".

6. Procédé selon la revendication 4, caractérisé en ce que la susdite séquence d'insertion contient deux fragments DNA HBV, normalement et respectivement contenus dans DNA HBV, de part et d'autre de son site EcoRI, orientés dans la même direction de transcription, l'un de ces fragments de DNA HBV contenant la séquence de nucléotides du gène S qui est apte à coder pour le polypeptide responsable des propriétés immunologiques de l'antigène HBs et l'autre desdits fragments étant suffisamment long pour qu'il soit susceptible d'inclure le promoteur du gène S, la queue du second fragment étant reliée à la tête de l'autre fragment au niveau de ce site EcoRI.

7. Procédé selon la revendication 4, caractérisé par des délétions dans les susdits ADN orientés dans ladite séquence d'insertion les rendant inaptes à coder pour les particules de Dane entières.

8. Procédé selon la revendication 4, caractérisé en ce que la séquence d'insertion est constituée par l'ADN du virus de l'hépatite virale B dont a été délétée une partie suffisante du gène codant pour l'antigène HBc pour que la production de ce dernier antigène par les cellules transformées soit rendue impossible.

9. Procédé selon la revendication 4, caractérisé en ce que la séquence d'insertion est formée de l'ADN du virus de l'hépatite virale B, dont a été délété le fragment délimité par les extrémités BglII au niveau des nucléotides 1986 et 2425 de DNA HBV.

10. Procédé selon la revendication 8, caractérisé en ce que la séquence d'insertion comprend, outre le gène S, et en amont dans le sens inverse de la transcription, un sous-fragment suffisamment long pour qu'y soit incluse une séquence TATATAA, à une distance correspondant à un nombre de nucléotides de l'ordre de 235 du site EcoRI de DNA HBV.

11. Procédé selon la revendication 10, caractérisé en ce que la séquence d'insertion comprend également en outre un sous-fragment en aval du gène S dans le sens de la transcription, ce fragment s'étendant dans le sens de la transcription au-delà du site BamHI, situé entre le gène S et le gène C.

12. Procédé selon la revendication 11, caractérisé en ce que la susdite séquence d'insertion comprend la totalité du gène codant pour l'ARN messager correspondant à l'antigène HBs.

13. Application des cellules eucaryotes transformées par le procédé selon l'une quelconque des revendications 1 à 12, à la fabrication des protéines susceptibles d'être codées par les fragments d'ADN contenus dans la séquence d'insertion susdite, par transformation d'une culture de cellules eucaryotes,

- 11

caractérisée en ce qu'elle comprend la récupération des protéines excrétées à partir du milieu de culture desdites cellules.

14. Séquence d'insertion pour son expression dans des cellules eucaryotes définies dans l'une quelconque des revendications 1 à 3, caractérisée en ce qu'elle est formée de l'ADN du virus de l'hépatite virale B, dont a été délété le fragment délimité par les extrémités BgIII au niveau des nucléotides 1986 et 2425 de DNA HBV.

15. Séquence d'insertion dérivée de l'ADN du virus de l'hépatite B pour son expression dans des cellules eucaryotes définies dans l'une quelconque des revendications 1 à 3, caractérisée en ce qu'elle comprend, outre le gène S, et en amont dans le sens inverse de la transcription, un sous-fragment suffisamment long pour qu'y soit incluse la séquence TATATAA, à une distance correspondant à un nombre de nucléotides de l'ordre de 235 du site EcoRI de DNA HBV et en ce qu'elle comporte une délétion complète du gène codant pour l'antigéne HBc.

16. Séquence d'insertion selon la revendication 15, caractérisée en ce qu'elle comprend également en outre un sous-fragment en aval du gène S dans le sens de la transcription, ce fragment s'étendant dans le sens de la transcription au-delà du site BamHI, situé entre le gène S et le gène C.

17. Séquence d'insertion selon la revendication 16, caractérisée en ce qu'elle comprend la totalité du gène codant pour l'ARN messager correspondant au gène S.

18. Vecteur modifié par une séquence d'insertion pour la transformation de cellules eucaryotes définies dans l'une quelconque des revendications 1 à 3, en vue de l'expression du gène S, caractérisé en ce que cette séquence d'insertion est conforme à celle définie dans l'une quelconque des revendications 14 à 17.

19. Vecteur pour la transformation de cellules eucaryotes pour la transformation de cellules eucaryotes définies dans l'une quelconque des revendications 1 à 3, en vue de l'expression du gène S, caractérisé en ce qu'il est modifié par une séquence d'insertion constituée par la totalité de DNA HBV, dont a été délétée une partie suffisante du gène codant pour l'antigène HBc, pour que la production de ce dernier antigène par les cellules infectées soit rendue impossible.

20. Vecteur selon la revendication 19, caractérisée en ce que sa séquence d'insertion contient deux fragments DNA HBV, normalement et respectivement contenus dans DNA HBV, de part et d'autre de son site EcoRI, orientés dans la même direction de transcription, l'un de ces fragments de DNA HBV contenant la séquence de nucléotides du gène S qui est apte à coder pour le polypeptide responsable des propriétés immunologiques de l'antigène HBs et l'autre desdits fragments étant suffisamment long pour qu'il soit susceptible d'inclure le promoteur du gène S, la queue du second fragment étant reliée à la tête de l'autre fragment au niveau de ce site EcoRI.

21. Vecteur pour la transformation de cellules eucaryotes, caractérisé en ce qu'il contient une séquence d'insertion contenant au moins deux DNA HBV orientés dans la même direction de transcription et dans laquelle la queue de l'un est reliée à la tête de l'autre.

22. Composition utilisable pour la constitution de vaccins, caractérisée en ce qu'elle consiste en des particules contenant des protéines, ayant les propriétés immunogéniques et immunologiques caractéristiques de l'antigène HBsAg, ayant un niveau de pureté total pour ce qui est de l'absence de toute particule de Dane, d'antigène HBc, de tout composant sérique d'origine humaine et d'ADN-polymérase, ces particules ayant été synthétisées par une culture de cellules eucaryotes transformées au préalable par un vecteur contenant le gène S du génome du virus de l'hépatite B.

23. Produit ayant des propriétés vaccinantes contre le virus de l'hépatite B, caractérisé:

— en ce qu'il consiste en des particules ayant des tailles de 18 à 25 nm et une densité permettant leur isolement dans une zone de densité de 1,20 g/ml dans un gradient de densité à base de CsCl;

— en ce que ces particules sont agglutinées par des anticorps qui agglutinent l'antigène HBsAg de sérum humain;

— en ce que ces particules sont capables d'induire *in vivo* la production d'anticorps actifs contre le virus de l'hépatite B;

— en ce qu'elles sont un degrée de pureté totale pour ce qui est des particules de Dane, des antigènes HBc et HBe qui sont totalement absents et

— en ce qu'elles sont exemptes de tout composant humain, notamment de protéines humaines.

24. Produit selon la revendication 22 ou 23, caractérisé par l'absence d'ADN-polymérase.

25. Produit selon l'une quelconque des revendications 22 à 24, caractérisé en ce que les particules sont sphériques.

26. Produit selon l'une quelconque des revendications 22 à 25 caractérisé en ce qu'il a été obtenu par le procédé selon l'une quelconque des revendications 1 à 12.

27. Cellules eucaryotes transformées par le procédé selon l'une quelconque des revendications 1 à 12, et capable de produire des particules, telles que définies dans l'une quelconque des revendications 22 à 26 dans leur milieu de culture.

28. Cellules eucaryotes transformées selon la revendication 27, caractérisées en ce qu'elles sont d'origine humaine et capables d'excréter lesdites particules sphériques dans leur milieu de culture.

29. Cellules eucaryotes transformées selon la revendication 27, caractérisées en ce qu'elles ont été obtenues à partir de cellules de souris et capables d'excréter lesdites particules sphériques dans leur milieu de culture.

30. Composition vaccinante active contre l'hépatite virale contenant le produit tel que défini dans l'une quelconque des revendications 22 à 26, en association avec un véhicule pharmaceutique approprié.

**Patentansprüche**

1. Verfahren zur Gewinning einer Kultur eukaryonitischer kompetenter Zellen, die fähig sind, DNA von Hepatitis verursachenden Viren zu exprimieren, wobei die Kompetenz der einzusetzenden eukaryontischen Zellen nach ihrer Fähigkeit beurteilt wira, nach der Transformation mit einem eine Tandemsequenz aus zwei HBV-DNA's enthaltenden Vektor ein immunogenes Protein zu sekretieren, das in vivo die Bildung aktiver Antikörper gegen den gesamten, Hepatitis B verursachenden Virus induziert, dadurch gekennzeichnet, daß man diese Kultur eukaryontischer Zellen mit einem Vektor, insbesondere einem Plasmid, transformiert, der selbst eine Insertionssequenz enthält, die mindestens den Teil der viralen DNA aufweist, der für ein immunogenes, in vivo die Bildung aktiver Antikörper gegen den Gesamtvirus induzierendes Protein codiert, und auch den Promoter des Virus, unter dessen Kontrolle die Transkription und Translation des vorgenannten Teils der Virus DNA im normalen, durch diesen Virus infizierten Wirt stattfindet.

2. Verfahren nach Anspruch 1, dadurch gekennzeichnet, daß die transformierten Zellen Mauszellen sind.

3. Verfahren nach Anspruch 1, dadurch gekennzeichnet, daß die transformierten Zellen menschliche Zellen sind.

4. Verfahren nach einem der Ansprüche 1 bis 3 zur Herstellung einer Kultur eukaryontischer Zellen, die das Gen S des Hepatitis B-Virus-Genoms exprimieren, dadurch gekennzeichnet, daß die vorgenannte Insertionssequenz von HBV-DNA stammt, und in der der für das immunogene Protein codierende Teil der viralen DNA die Nucleotidsequenz des Gens S enthält, die für das die immunologischen Eigenschaften des HBs-Antigens tragende Polypeptid codiert, wobei diesem Teil oder Fragment der viralen DNA in Richtung der Transkription ein Fragment der HBV-DNA vorausgeht, das genügend lang ist, um den Promoter des Gens S enthalten zu können.

5. Verfahren nach einem der Ansprüche 1 bis 3, dadurch gekennzeichnet, daß die vorgenannte Insertionssequenz aus zwei Einheiten HBV-DNA besteht, die eine Tandem-Sequenz bilden.

6. Verfahren nach Anspruch 4, dadurch gekennzeichnet, daß die vorgenannte Insertionssequenz auf beiden Seiten ihrer EcoRI-Stelle zwei HBV-DNA-Fragmente enthält, die jeweils normalerweise in HBV-DNA enthalten sind, und die in der gleichen Transkriptionsrichtung ausgerichtet sind, wobei eines dieser HBV-DNA-Fragmente die Nucleotidsequenz des Gens S enthält, die zur Codierung des Polypeptids geeignet ist, das dem HBs-Antigen die immunologischen Eigenschaften verleiht, und das andere dieser Fragmente genügend lang ist, damit es den Promoter des Gens S einschließen kann, wobei der Schwanz des zweiten Fragmentes mit dem Kopf des anderen Fragmentes an der EcoRI-Stelle verknüpft ist.

7. Verfahren nach Anspruch 4, gekennzeichnet durch Deletionen in den vorgenannten DNA's, die so in der genannten Insertionssequenz angeordnet sind, daß diese nicht mehr fähig sind, für die vollständigen Danepartikel zu codieren.

8. Verfahren nach Anspruch 4, dadurch gekennzeichnet, daß die Insertionssequenz aus Hepatitis B-Virus-DNA besteht, in der ein genügend großer Teil des Gens, das für das HBc-Antigen codiert, deletiert ist, so daß die Bildung des letzteren Antigens durch die transformierten Zellen nicht mehr möglich ist.

9. Verfahren nach Anspruch 4, dadurch gekennzeichnet, daß die Insertionssequenz aus Hepatitis B-Virus-DNA besteht, in der das Fragment der HBV-DNA, das an den Nucleotiden 1986 und 2425 durch BglII-Schnittstellen begrenzt ist, deletiert ist.

10. Verfahren nach Anspruch 8, dadurch gekennzeichnet, daß die Insertionssequenz außer dem Gen S, stromaufwärts in umgekehrter Transkriptionsrichtung ein Unterfragment enthält, des genügend lang ist, um die Sequenz TATATAA einzuschließen, in einem Abstand zur EcoRI-Stelle der HBV-DNA, der einer Zahl von Nucleotiden in der Größenordnung von 235 entspricht.

11. Verfahren nach Anspruch 10, dadurch gekennzeichnet, daß die Insertionssequenz außerdem ein Unterfragment stromabwärts zum Gen S in Transkriptionsrichtung enthält, wobei dieses Fragment sich in Transkriptionsrichtung über die BamHI-Stelle hinaus erstreckt, die zwischen dem Gen S und dem Gen C liegt.

12. Verfahren nach Anspruch 11, dadurch gekennzeichnet, daß die vorgenannte Insertionssequenz das gesamte Gen enthält, das für die m-RNA des HBs-Antigens codiert.

13. Verwendung der transformierten eukaryontischen Zellen für das Verfahren nach einem der Ansprüche 1 bis 12, zur Herstellung von Proteinen, die durch die in der vorgenannten Insertionssequenz enthaltenen DNA-Fragments codiert werden können, durch Transformation einer Kultur eukaryontischer Zellen, dadurch gekennzeichnet, daß man die sekretierten Proteine aus der Kulturflüssigkeit der Zellen gewinnt.

14. Insertionssequenz zur Expression in den eukaryontischen Zellen nach einem der Ansprüche 1 bis 3, dadurch gekennzeichnet, daß sie aus Hepatitis B-Virus-DNA besteht, in der das Fragment der HBV-DNA, das bei den Nucleotiden 1986 und 2425 durch BglII-Schnittstellen begrenzt ist, deletiert ist.

13

**0 038 765**

15. Insertionssequenz aus der Hepatitis B-Virus-DNA zur Expression in den eukaryontischen Zellen gemäß einem der Ansprüche 1 bis 3, dadurch gekennzeichnet, daß sie außer dem Gen S, stromaufwärts in umgekehrter Transkriptionsrichtung ein Unterfragment enthält, das genügend lang ist, um die Sequenz TATATAA einzuschließen, in einem Abstand zur EcoRI-Schnittstelle der HBV-DNA, der einer Zahl der Nucleotide in der Größenordnung von 235 entspricht, und daß sie eine vollständige Deletion des Gens aufweist, das für das Antigen HBc codiert.

16. Insertionssequenz nach Anspruch 15, dadurch gekennzeichnet, daß sie außerdem ein Unterfragment stromabwärts vom Gen S in Transkriptionsrichtung enthält, wobei dieses Fragment sich in Transkriptionsrichtung über die BamHI-Stelle hinaus erstreckt, die zwischen dem Gen S und dem Gen C liegt.

17. Insertionssequenz nach Anspruch 16, dadurch gekennzeichnet, daß sie das gesamte Gen enthält, das für die m-RNA des Gens S codiert.

18. Vektor, modifiziert durch eine Insertionssequenz für die Transformation der eukaryontischen Zellen gemäß einem der Ansprüche 1 bis 3, zur Expression des Gens S, dadurch gekennzeichnet, daß diese Insertionssequenz einer Sequenz gemäß einem der Ansprüche 14 bis 17 entspricht.

19. Vektor für die Transformation eukaryontischer Zellen gemäß einem der Ansprüche 1 bis 3, zur Expression des Gens S, dadurch gekennzeichnet, daß er durch einen Insertionssequenz aus der gesamten HBV-DNA modifiziert ist, wobei ein genügend großer Teil des für das Antigen HBc codierenden Gens deletiert ist, so daß die Bildung des letzteren Antigens durch die infizierten Zellen unmöglich ist.

20. Vektor nach Anspruch 19, dadurch gekennzeichnet, daß seine Insertionssequenz auf beiden Seiten ihrer EcoRI-Stelle zwei HBV-DNA-Fragmente enthält, die jeweils normalerweise in HBV-DNA enthalten sind, und die in die gleiche Richtung der Transkription ausgerichtet sind, wobei eines dieser HBV-DNA-Fragmente die Nucleotidsequenz des Gens S enthält, die zur Codierung des Polypeptids geeignet ist, das für die immunologischen Eigenschaften des Antigens HBs verantwortlich ist, und wobei das andere dieser Fragmente genügend lang ist, um den Promotor des Gens S aufnehmen zu können, wobei der Schwanz des zweiten Fragments mit dem Kopf des anderen Fragments an der EcoRI-Stelle verknüpft ist.

21. Vektor zur Transformation eukaryontischer Zellen, dadurch gekennzeichnet, daß er eine Insertionssequenz enthält, die zwei in der gleichen Transkriptionsrichtung ausgerichtete HBV DNA-Fragmente enthält, und in der der Schwanz des einen mit dem Kopf des anderen verknüpft ist.

22. Mittel für die Herstellung von Impfstoffen, dadurch gekennzeichnet, daß es aus Partikeln besteht, die Proteine mit immunogenen und immunologischen Eigenschaften, die für das Antigen HBsAg charakteristisch sind, aufweisen, wobei sie vollständig rein sind, so daß sie keine ganzen Dane-Partikel, HBc-Antigen, menschliche Serumbestandteile und DNA-Polymerase mehr aufweisen, wobei diese Partikel durch eine Kultur eukaryontischer Zellen synthetisiert wurden, die vorher mit einem Vektor, der das Gen S des Genoms des Hepatitis B-Virus aufweist, transformiert wurden.

23. Produkt, geeignet als Impfstoff gegen den Hepatitis B-Virus, mit folgenden Eigenschaften: bestehend aus Partikeln, die eine Größe von 18 bis 25 nm aufweisen und eine Dichte, die es erlaubt, sie in einer Dichtezone von 1,20 g/ml in einem Cäsiumchlorid-Dichtegradienten zu isolieren; diese Partikel werden durch Antikörper agglutiniert, die das Antigen HBsAg des menschlichen Serums agglutinieren; diese Partikel können in vivo die Produktion aktiver Antikörper gegen den Hepatitis B-Virus induzieren; sie haben einen solchen Grad der Reinheit, daß Dane-Partikel, HBc- und HBe-Antigene vollständig abwesend sind; und sie sind frei von menschlichen Komponenten, insbesondere von menschlichen Proteinen.

24. Produkt nach den Ansprüchen 22 oder 23, gekennzeichnet durch die Abwesenheit von DNA-Polymerase.

25. Produkt nach einem der Ansprüche 22 bis 24, gekennzeichnet durch sphärische Partikel.

26. Produkt nach einem der Ansprüche 22 bis 25, dadurch gekennzeichnet, daß es nach einem Verfahren nach einem der Ansprüche 1 bis 12 erhalten wurde.

27. Eukaryontische Zellen, die nach einem Verfahren nach einem der Ansprüche 1 bis 12 transformiert wurden, und die fähig sind, in ihr Kulturmedium Partikel nach einem der Ansprüche 22 bis 26 zu produzieren.

28. Eukaryontische transformierte Zellen nach Anspruch 27, dadurch gekennzeichnet, daß sie menschlichen Ursprungs sind und die genannten sphärischen Partikel in ihr Kulturmedium sekretieren können.

29. Eukaryontische transformierte Zellen nach Anspruch 27, dadurch gekennzeichnet, daß sie aus Mäusezellen erhalten wurden und fähig sind, die vorgenannten sphärischen Partikel in ihr Kulturmedium zu sekretieren.

30. Impfstoff gegen virale Hepatitis, enthaltend das Produkt nach einem der Ansprüche 22 bis 26, zusammen mit einem pharmakologisch verträglichen Träger.

**Claims**

1. A process for producing a culture of competent eucaryotic cells capable of expressing a viral DNA and selected from those which are responsible for a hepatitis, wherein the competence of the eucaryotic

14

**0 038 765**

cells which are capable of being used can be appreciated by the capacity which can be imparted thereto, after transformation thereof with a vector containing a tandem sequence comprising two DNA HBV, to secrete an immunogenic protein capable of inducing *in vivo* the production of active antibodies against the entire virus of viral hepatitis B, characterised by the operation which consists of transforming said culture of eucaryotic cells with a vector, more particularly a plasmid which itself contains an insertion sequence which itself comprises at least the part of the viral DNA which codes for an immunogenic protein capable of inducing *in vivo* the production of active antibodies with regard to the entire virus, and the promoter of said virus, under the control of which are effected transcription and translation in the normal host infected by said virus of said part of viral DNA.

2. A process according to claim 1 characterised in that the transformed cells are mouse cells.

3. A process according to claim 1 characterised in that the transformed cells are cells of human origin.

4. A process according to any one of claims 1 to 3 applied to the production of a culture of eucaryotic cells expressing the gene S of the genome of the virus of hepatitis B, characterised in that said insertion sequence is derived from DNA HBV, that the part of the viral DNA which codes for the immunogenic protein contains the sequence of nucleotides of the gene S which codes for the polypeptide having the immunological properties of the antigen HBs, said part or fragment of the viral DNA being preceded, with respect to the direction of transcription, by a fragment of DNA HBV which is sufficiently long for it to be capable of including the promoter of the gene S.

5. A process according to any one of claims 1 to 3 characterised in that said insertion sequence is formed by two units of DNA HBV forming a 'tandem' sequence.

6. A process according to claim 4 characterised in that said insertion sequence contains two DNA HBV fragments which are normally and respectively contained in DNA HBV, on respective sides of its site EcoRI, which are oriented in the same transcription direction, one of said fragments of DNA HBV containing the sequence of nucleotides of the gene S which is capable of coding for the polypeptide responsible for the immunological properties of the antigen HBs and the other of said fragments being sufficiently long for it to be capable of including the promoter of the gene S, the tail of the second fragment being joined to the head of the other fragment at the location of said site EcoRI.

7. A process according to claim 4 characterised by deletions in said DNAs oriented within said insertion sequence making them unsuitable for coding for the entire Dane particles.

8. A process according to claim 4 characterised in that the insertion sequence is formed by the DNA of the virus of viral hepatitis B from which there has been deleted a sufficient part of the gene which codes for the antigen HBc for the production of said latter antigen by the transformed cells to be made impossible.

9. A process according to claim 4 characterised in that the insertion sequence is formed by the DNA of the virus of viral hepatitis B from which there has been deleted the fragment delimited by BglII ends at the position of the nucleotides 1986 and 2425 of DNA HBV.

10. A process according to claim 8 characterised in that the insertion sequence comprises, besides the gene S and upstream in the reverse direction to transcription, a sub-fragment sufficiently long for it to include a sequence TATATAA, at a distance corresponding to a number of nucleotides of the order of 235 from the site EcoRI of DNA HBV.

11. A process according to claim 10 characterised in that the insertion sequence also comprises a sub-fragment downstream of the gene S in the direction of transcription, said fragment extending in the direction of transcription beyond the site BamHI, disposed between the gene S and the gene C.

12. A process according to claim 11 characterised in that said insertion sequence comprises the whole of the gene which codes for the messenger RNA corresponding to the antigen HBs.

13. Use of the eucaryotic cells transformed by the process according to any one of claims 1 to 12 for the production of proteins capable of being coded by the fragments of DNA which are contained in said insertion sequence, by transformation of a culture of eucaryotic cells, characterised in that it comprises the recovery of the proteins which are excreted from the culture medium of said cells.

14. An insertion sequence for expression thereof in eucaryotic cells as defined in any one of claims 1 to 3 characterised in that it is formed by the DNA of the virus of viral hepatitis B from which there has been deleted the fragment delimited by BglII ends at the position of the nucleotides 1986 and 2425 of DNA HBV.

15. An insertion sequence derived from the DNA of the virus of hepatitis B for expression thereof in eucaryotic cells as defined in any one of claims 1 to 3 characterised in that, besides the gene S and upstream in the opposite direction to transcription, it comprises a sub-fragment sufficiently long for it to include the sequence TATATAA, at a distance corresponding to a number of nucleotides of the order of 235 from the site EcoRI of DNA HBV, and that it includes a complete deletion of the gene which codes for the antigen HBc.

16. An insertion sequence according to claim 15 characterised in that it also comprises a sub-fragment downstream of the gene S in the direction of transcription, said fragment extending in the direction of transcription beyond the site BamHI, disposed between the gene S and the gene C.

17. An insertion sequence according to claim 16 characterised in that it comprises the whole of the gene which codes for the messenger RNA corresponding to the gene S.

18. A vector modified by an insertion sequence for the transformation of eucaryotic cells as defined in any one of claims 1 to 3, for expression of the gene S, characterised in that said insertion sequence is in accordance with that defined in any one of claims 14 to 17.

15

19. A vector for the transformation of eucaryotic cells for the transformation of eucaryotic cells as defined in any one of claims 1 to 3, for expression of the gene S, characterised in that it is modified by an insertion sequence formed by the whole of DNA HBV from which has been deleted a sufficient part of the gene which codes for the antigen HBc, for the production of the latter antigen by the infected cells to be made impossible.

20. A vector according to claim 19 characterised in that the insertion sequence contains two DNA HBV fragments which are normally and respectively contained in DNA HBV on respective sides of its site EcoRl, which are oriented in the same transcription direction, one of said fragments of DNA HBV containing the sequence of nucleotides of the gene S which is capable of coding for the polypeptide responsible for the immunological properties of the antigen HBs and the other of said fragments being sufficiently long for it to be capable of including the promoter of the gene S, the tail of the second fragment being joined to the head of the other fragment at the location of said site EcoRl.

21. A vector for the transformation of eucaryotic cells characterised in that it contains an insertion sequence containing at least two DNA HBV which are oriented in the same transcription direction, in which the tail of one is joined to the head of the other.

22. A composition which can be used for making vaccines characterised in that it consists of particles containing proteins, having the immunogenic and immunological properties characteristic of the antigen HBsAg, having a total level of purity as regards the absence of any Dane particle, antigen HBc, any serum component of human origin and DNA-polymerase, said particles having been synthesised by a culture of eucaryotic cells previously transformed by a vector containing the gene S of the genome of the virus of hepatitis B.

23. A product having vaccinating properties against the virus of hepatitis B, characterised:

— in that it consists of particles having sizes of from 18 to 25 nm and a density permitting isolation thereof in a density region of 1.20 g/ml in a CsCl-based density gradient;
— in that said particles are agglutinated by antibodies which agglutinate the antigen HBsAg of human serum;
— in that said particles are capable of inducing *in vivo* the production of active antibodies against the virus of hepatitis B;
— in that they have a degree of total purity as regards Dane particles, and antigens HBc and HBe which are totally absent, and
— in that they are free of any human component, in particular human proteins.

24. A product according to claim 22 or claim 23 characterised by the absence of DNA-polymerase.

25. A product according to any one of claims 22 to 24 characterised in that the particles are spherical.

26. A product according to any one of claims 22 to 25 characterised in that it was produced by the process according to any one of claims 1 to 12.

27. Eucaryotic cells transformed by the process according to any one of claims 1 to 12 and capable of producing particles, as defined in any one of claims 22 to 26 in their culture medium.

28. Transformed eucaryotic cells according to claim 27 characterised in that they are of human origin and are capable of excreting said spherical particles in their culture medium.

29. Transformed eucaryotic cells according to claim 27 characterised in that they were produced from cells of mice and are capable of excreting said spherical particles in their culture medium.

30. An active vaccinating composition against viral hepatitis containing the product as defined in any one of claims 22 to 26, in association with a suitable pharmaceutical carrier.

Fig.1.

Fig.2a.

Fig.2b.

Fig.2c.